(19) Europäisches Patentamt
European Patent Office
Office européen des brevets

(11) Publication number: **0 269 656 B1**

(12) **EUROPEAN PATENT SPECIFICATION**

(45) Date of publication of patent specification: **29.12.93**

(51) Int. Cl.5: **C07C 233/03**, C07C 233/16, C07C 233/57, C07C 69/38, C07C 69/74, C07C 327/20, C07D 521/00, A01N 37/30, A01N 37/04, A01N 37/08

(21) Application number: **87902948.6**

(22) Date of filing: **30.03.87**

(86) International application number: **PCT/US87/00649**

(87) International publication number: **WO 87/05898 (08.10.87 87/22)**

The file contains technical information submitted after the application was filed and not included in this specification

# (54) NOVEL MALONIC ACID DERIVATIVE COMPOUNDS AND COMPOSITIONS THEREOF FOR RETARDING PLANT GROWTH.

(30) Priority: **31.03.86 US 846670**
**06.03.87 US 18129**

(43) Date of publication of application: **08.06.88 Bulletin 88/23**

(45) Publication of the grant of the patent: **29.12.93 Bulletin 93/52**

(84) Designated Contracting States: **AT BE CH DE FR GB IT LI NL SE**

(56) References cited:
**WO-A-87/05897**
**GB-A- 1 086 326**
**US-A- 3 072 473**

**CHEMICAL ABSTRACTS, vol. 82, no. 25, 23 June 1975, Columbus, Ohio, US; page 463, abstract no. 170074n & JP-A-74 116 019**

(73) Proprietor: **RHONE POULENC NEDERLAND B.V.**
**Draaistroom 1**
**Postbus 10**
**NL-1180 AA Amstelveen(NL)**

(72) Inventor: **MANNING, David, Treadway**
**102 Summerwinds Drive**
**Cary, NC 27511(US)**
Inventor: **CAPPY, James, Joseph**
**4905 Kaplan Drive**
**Raleigh, NC 27606(US)**
Inventor: **SEE, Raymond, Michael**
**Rural Route 1 - Box 132B**
**Franklinton, NC 27525(US)**
Inventor: **COOKE, Anson, Richard**
**1210 Huntsman Drive**
**Durham, NC 27713(US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.6/3.3.1)

CHEMICAL ABSTRACTS, vol. 68, no. 17, 22 April 1968, Columbus, Ohio, US; A. BARUFFINI et al, "Malonanilic acids and derivatives", page 7513, abstract no. 77925s

CHEMICAL ABSTRACTS, vo. 104, no. 9, 3 March 1966, Columbus, Ohio, US; page 659, abstract no. 68620y & JP-A-60 166 651

IL FARMACO, Edizione Scientifica, vol. 31, no. 7, July 1976; K. A. ZIRVI et al, "Diamides of cyclobutane-1,1-dicarboxylic acid", pages 546-548

CHEMICAL ABSTRACTS, vol. 105, no. 15, 13 October 1986, Columbus, Ohio, US; S. NOBORU et al, "Plant growth regulating activities of ethoxycarbonylacetanilides", abstract no. 129320v

CHEMICAL ABSTRACTS, vol. 101, no. 7, 13 August 1984, Columbus, Ohio, US; page 206, abstract no. 50143y & JP-A-593 9803

CHEMICAL ABSTRACTS, vol. 102, no. 13, 1 April 1985, Columbus, Ohio, US; S. NOBORU et al, "Plant growth regulating properties of some substituted malonyl monoanilides", page 247, abstract no. 108089z

Inventor: **FRITZ, Charles, David**
**8717 Wellsley Way**
**Raleigh, NC 27612(US)**
Inventor: **WHEELER, Thomas, Neil**
**8605 Woodlawn Drive**
**Raleigh, NC 27612(US)**

(74) Representative: **Trolliet, Maurice et al**
**Rhône-Poulenc Agrochimie**
**Département Propriété Industrielle**
**14-20 Rue Pierre Baizet**
**B.P. 9163**
**F-69263 Lyon Cedex 09 (FR)**

## Description

This invention relates to novel malonic acid derivative compounds and to the compositions thereof, for retarding plant growth.

### Background of the Invention

Certain malonic acid derivative compounds have been known for some time in the art. See, for example, U.S. Patent 2,504,896 and U.S. Patent 3,254,108. Some malonic acid derivative compounds have been described in the art as capable of providing certain plant growth regulating responses such as prevention of fruit drop, rooting of cuttings and formation of parthenogenetic fruit.

U.S. Patent 3,072,473 describes N-arylmalonamic acids and their esters and salts, N, N'-diarylmalonamides, N-alkyl-N-arylmalonamic acids and their esters and salts, and N, N'-dialkyl-N, N'-diarylmalonamides which may be useful as plant growth regulants and herbicides. Japanese Patent 84 39,803 (1984) describes malonic acid anilide derivative compounds which may be useful as plant growth regulators. The plant growth regulating properties of substituted malonyl monoanilides are described by Shindo, N. and Kato, M., Meiji Daigaku Noogakubu Kenkyu Hokoku, Vol. 63, pp. 41-58 (1984).

However, certain malonic acid derivative compounds and compositions thereof, for retarding plant growth as described herein, have not been disclosed in the art.

Accordingly, it is an object of this invention to provide novel malonic acid derivative compounds and compositions thereof. These and other objects will readily become apparent to those skilled in the art in light of the teachings herein set forth.

### Disclosure of the Invention

This invention relates to novel malonic acid derivative compounds. More precisely this invention relates to a cyclopropylmalonanilate derivative compound capable of retarding plant growth having the formula:

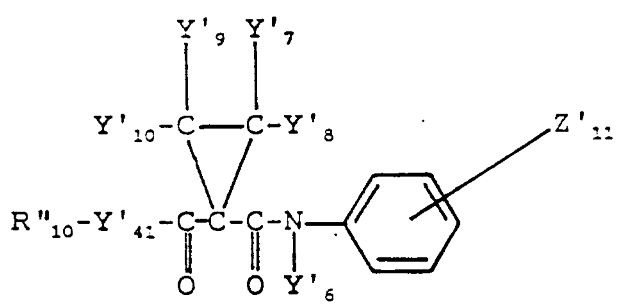

1

wherein $Z'_{11}$, $Y'_6$, $Y'_7$, $Y'_8$, $Y'_9$, $Y'_{10}$, $Y'_{41}$ and $R''_{10}$ are defined hereinafter.

This invention further relates to compositions for retarding plant growth comprising an acceptable carrier and an effective amount, sufficient to retard plant growth, of a compound of formula 1.

### Detailed Description

As indicated above, this invention relates in a first object to a novel cyclopropylmalonanilate derivative compound. More precisely, this invention relates to a cyclopropylmalonanilate derivative compound having the formula:

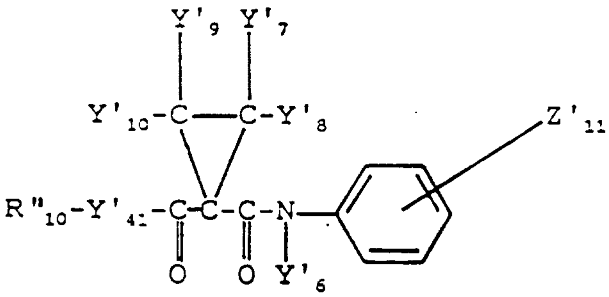

1

wherein:

$Z'_{11}$ is the same or different and is one or more hydrogen, halogen, haloalkyl, polyhaloalkyl, polyhaloalkoxy, alkyl, alkoxy, alkylthio, alkylsulfonyl, alkylsulfinyl, aryl, aryloxy, arylthio, arylsulfonyl, nitro, cyano, dialkoxyphosphinyl, alkanoyl, aroyl, alkoxycarbonyl, alkoxycarbonylalkyl, alkanoylamino, sulfonylamino, alkylsulfonylamino, alkanoyloxy, alkenyl or -CH = CH-CH = CH-;

$Y'_6$ is hydrogen or alkyl;

$Y'_7$, $Y'_8$, $Y'_9$ and $Y'_{10}$ are independently hydrogen, halogen or alkyl;

$Y'_{41}$ is 0, S, or NH;

$R''_{10}$ is hydrogen, ammonium, alkylammonium, polyalkylammonium, hydroxyalkylammonium, poly-(hydroxyalkyl)ammonium, an alkali metal or alkaline earth metal or alkyl, hydroxyalkyl, alkoxyalkyl, alkoxycarbonylalkyl, alkylaminoalkyl, dialkylaminoalkyl, aryl, mercaptoalkyl, alkylthioalkyl, arylthioalkyl, aryloxyalkyl, alkylsylfonylalkyl, alkylsulfinylalkyl, alkanoylalkyl, aroylalkyl, dialkoxyphosphinylalkyl, diaryloxyphosphinylalkyl, hydroxyalkylthioalkyl, hydroxyalkylsulfonylalkyl, alkoxyalkylthioalkyl, alkoxyalkylsulfonylalkyl, poly(oxyalkylene)alkyl, cyanoalkyl, nitroalkyl, alkylideneamino, carbamoylalkyl, alkylcarbamoylalkyl, dialkylcarbamoylalkyl, aminoalkyl, alkanoylaminoalkyl, alkanoyloxyalkyl, alkoxycarbonylaminoalkyl, cyanoaminoalkyl, carbamoyloxyalkyl, alkylcarbamoyloxyalkyl, dialkylcarbamoyloxyalkyl, alkoxycarbonyloxyalkyl, alkoxycarbonylthioalkyl, aminosulfonylalkyl, alkylaminosulfonylalkyl or dialkylaminosulfonylalkyl.

Illustrative cyclopropylmalonanilate derivative compounds within the scope of formula 1 which may be prepared and used for retarding plant growth according to the present invention are included in Tables 1 and 2 below.

TABLE 1

Representative Malonic Acid Derivative Compounds

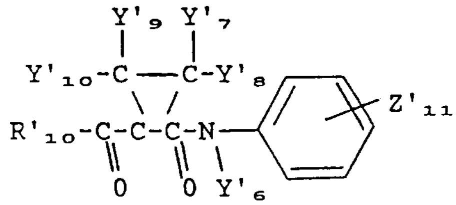

| $Y'_7$ | $Y'_8$ | $Y'_9$ | $Y'_{10}$ | $Y'_6$ | $R'_{10}=R''_{10}-Y'_{41}$ | $Z'_{11}$ |
|---|---|---|---|---|---|---|
| H | H | H | H | H | OH | $2-C_2H_5-4-Cl$ |
| H | H | H | H | H | $OCH_3$ | $2-C_2H_5-4-Cl$ |
| H | H | H | H | H | OH | $2-C_2H_5-4-Br$ |
| H | H | H | H | H | $OC_2H_5$ | $2-C_2H_5-4-Br$ |
| H | H | H | H | $CH_3$ | OH | 4-Cl |
| H | H | H | H | $CH_3$ | ONa | 4-Br |
| H | H | H | H | $CH_3$ | OH | $3,4-Cl_2$ |
| H | H | H | H | $CH_3$ | $OC_2H_5$ | 2-Cl-4-Br |
| H | H | H | H | $CH_3$ | OH | 2-F-4-Br |
| H | H | H | H | H | OH | 3-F-4-Cl |
| H | H | H | H | H | $OCH_3$ | 3-F-4-Br |
| H | H | H | H | H | $NH_2$ | $2-CH_3-4-Br$ |
| H | H | H | H | H | $ONH_4$ | $2-CH_3-4-Br$ |
| H | H | $CH_3$ | H | H | OH | 2-F-4-Cl |
| Cl | Cl | $CH_3$ | H | H | OK | $2-CH_3-4-Cl$ |

TABLE 1 (Continued)

Representative Malonic Acid Derivative Compounds

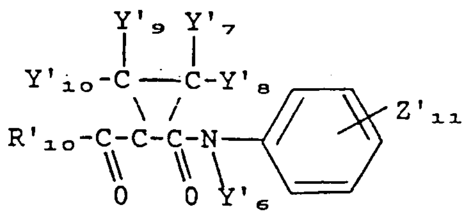

| $Y'_7$ | $Y'_8$ | $Y'_9$ | $Y'_{10}$ | $Y'_6$ | $R'_{10}=R''_{10}-Y'_{41}$ | $Z'_{11}$ |
|---|---|---|---|---|---|---|
| H | H | $CH_3$ | $CH_3$ | H | $OC_2H_5$ | 2,4-$Cl_2$ |
| $CH_3$ | $CH_3$ | $CH_3$ | $CH_3$ | H | ONa | 2-$CH_3$-4-Br |
| $CH_3$ | H | H | H | H | OH | 4-Cl |
| $CH_3$ | $CH_3$ | H | H | H | $ONH_4$ | 2-$C_2H_5$-4-Cl |
| Cl | H | H | H | H | OH | 2-F-4-Br |
| Cl | Cl | H | H | H | $OCH_3$ | 2,4-$Br_2$ |
| Cl | Cl | $CH_3$ | $CH_3$ | H | OH | 4-Cl |
| H | H | H | H | H | OH | 2-$CH_3$O-4,5-$Cl_2$ |
| H | H | H | H | H | OH | 2-$CH_3$O-3,5-$Cl_2$ |
| H | H | H | H | H | ONa | 2-$CF_3$-4-Cl |
| H | H | H | H | H | $OCH_3$ | 2-$CF_3$O-4-$CF_3$ |
| H | H | H | H | H | OK | 2-F-4-I |
| H | H | H | H | H | OH | 3-Br-4-Cl |
| $C_2H_5$ | H | H | H | H | OH | 2-$CH_3$-4-Br |
| H | H | $CH_3$ | $CH_3$ | H | $OC_2H_5$ | 2,4-$Cl_2$ |
| $CH_3$ | $CH_3$ | $CH_3$ | $CH_3$ | H | ONa | 2-$CH_3$-4-Br |
| Cl | Cl | $CH_3$ | $CH_3$ | H | OH | 4-Cl |

## TABLE 2

### Representative Malonic Acid Derivative Compounds

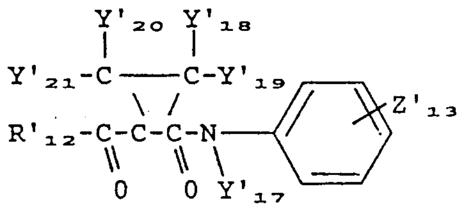

| $Y'_{18}$ | $Y'_{19}$ | $Y'_{20}$ | $Y'_{21}$ | $Y'_{17}$ | $Z'_{13}$ | $R'_{12}=R''_{10}-Y'_{41}$ |
|---|---|---|---|---|---|---|
| H | H | H | H | H | $2-CH_3-4-Br$ | $CH_2CH_2OCH_3$ |
| H | H | H | H | H | $2-CH_3-4-Br$ | $OCH_2CH_2OH$ |
| H | H | H | H | H | 4-Cl | $OCH_2CH_2SO_2CH_3$ |
| H | H | H | H | H | 2-Cl-4-Br | $OCH_2COCH_3$ |
| H | H | H | H | H | 2-F-4-Br | $OCH(CH_3)C{\equiv}N$ |
| H | H | H | H | H | $2-CH_3-4-Br$ | $OCH_2CH_2SO_2CH_2OH$ |
| H | H | H | H | $CH_3$ | $3,4-Br_2$ | $OCH_2CH_2OH$ |
| $CH_3$ | $CH_3$ | $CH_3$ | H | H | 4-Br | $O-N=CHCH_3$ |
| Cl | Cl | $CH_3$ | H | H | $2,4-Cl_2$ | $OCH_2CONH_2$ |
| H | H | $CH_3$ | $CH_3$ | $CH_3$ | 4-Cl | $SCH_3$ |
| $CH_3$ | H | $CH_3$ | $CH_3$ | H | 3-F-4-Cl | $SCH_2CO_2CH_3$ |
| Cl | Cl | $CH_3$ | $CH_3$ | H | $2-CF_3-4-Br$ | $OCH_2CH_2OH$ |
| H | H | H | H | H | $2,4-Br_2$ | $OCH_2CH_2OCH_2CH_2OH$ |
| H | H | H | H | H | 3-Br-4-Cl | $SC_6H_5$ |
| H | H | H | H | H | $2-CF_3O-4-$ | $OCH_2CH_2OH$ |
| H | H | H | H | H | $2-CF_3-4-I$ | $OCH_2COCH_3$ |

It is appreciated that the particular compounds listed in tables 1 and 2 hereinabove are illustrative of cyclopropylmalonanilate derivative compounds which can be used for retarding plant growth according to this invention. This invention is not to be construed as being limited only to these compounds; but rather, this invention includes the cyclopropylmalonanilate derivative compounds encompassed within formula 1 hereinabove.

The cyclopropylmalonanilate derivative compounds encompassed within formula 1 and the intermediate compounds used in the preparation thereof can be prepared by conventional methods known in the art and many may be available from various suppliers. More precisely, the cyclopropylmalonanilate derivative compounds can be prepared by the following general reaction scheme:

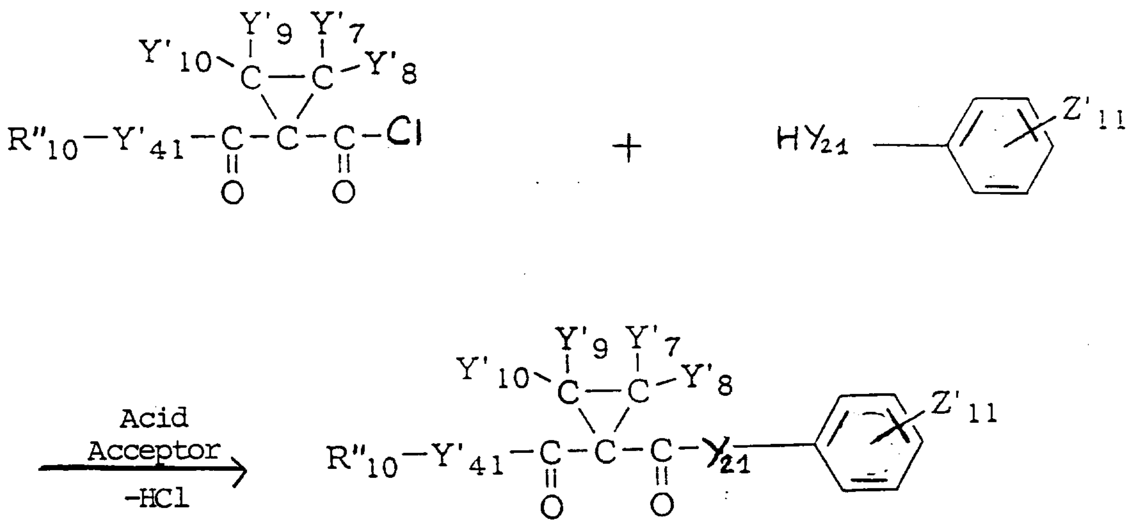


wherein $Z'_{11}$, $Y'_7$, $Y'_8$, $Y'_9$, $Y'_{10}$, $Y'_{41}$ and $R''_{10}$ are as defined hereinabove and wherein $Y_{21}$ is NH or N-(alkyl).

Reactions of this general type for preparing malonic acid derivative compounds including process conditions are described for example by Richter, G.H., Textbook of Organic Chemistry, Third Edition, John Wiley and Sons, New York, p. 486, according to the known Schotten Baumann procedure.

Specific cyclopropylmalonanilate derivative compounds which are prepared and used in a preferred embodiment of this invention include:

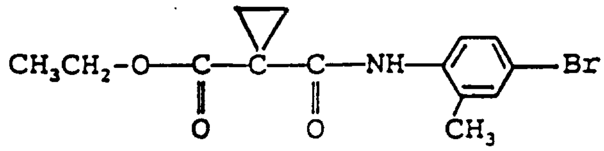

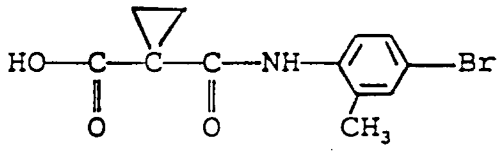

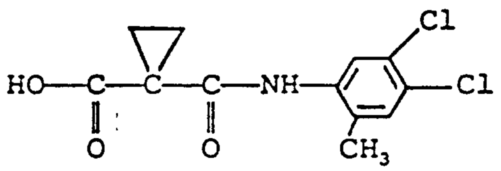

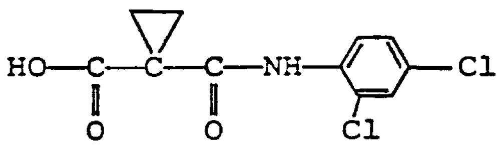

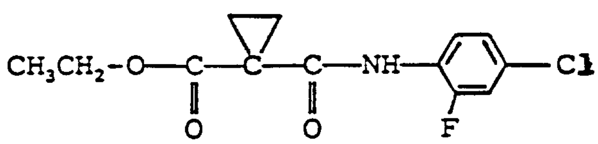

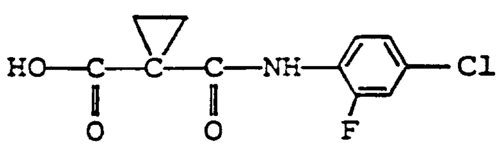

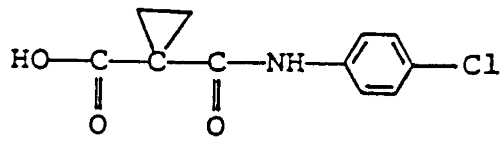

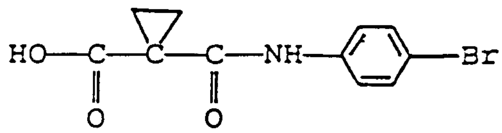

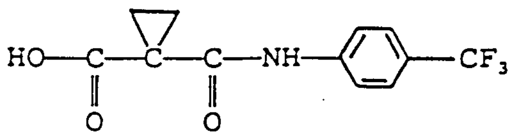

The international application published under the P.C.T. with the number 87/05781, on 8 October 1987 describes synergistic plant growth regulator compositions containing (i) an ethylene response or an ethylene-type response inducing agent and (ii) a malonic acid derivative compound of formula 1.

The cyclopropylmalonanilate derivative compounds of formula 1 have been found to significantly retard plant growth in comparison with untreated plants at similar conditions. In addition, the cyclopropylmalonanilate derivative compounds used in this invention are substantially non-phytotoxic to growing plants.

As used herein, an effective amount of a cyclopropylmalonanilate derivative compound for retarding plant growth refers to a growth retarding effective amount of the compound sufficient to retard plant growth. The effective amount of compound can vary over a wide range depending on the particular compound employed, the particular plant to be treated, environmental and climatic conditions, and the like. The amount of compound used preferably does not cause substantial phytotoxicity, e.g., foliar burn, chlorosis or necrosis, to the plant. In general, the compound can preferably be applied to plants at a concentration of from about 0.01 to 15 pounds of compound per acre as more fully described below.

The cyclopropylmalonanilate derivative compounds contemplated by formula 1 can be employed according to a variety of conventional methods known to those skilled in the art. Compositions containing the compounds as the active ingredient will usually comprise a carrier and/or diluent, either liquid or solid. Suitable liquid diluents or carriers include water, petroleum distillates, or other liquid carriers with or without surface active agents. Liquid concentrates can be prepared by dissolving one of these compounds with a nonphytotoxic solvent such as acetone, xylene, nitrobenzene, cyclohexanone or dimethyl formamide and dispersing the active ingredients in water with the aid of suitable surface active emulsifying and dispersing agents.

The choice of dispersing and emulsifying agents and the amount employed are dictated by the nature of the composition and the ability of the agent to facilitate the dispersion of the active ingredient. Generally, it is desirable to use as little of the agent as is possible, consistent with the desired dispersion of the active ingredient in the spray so that rain does not re-emulsify the active ingredient after it is applied to the plant and wash it off the plant. Nonionic, anionic, or cationic dispersing and emulsifying agents may be employed, for example, the condensation products of alkylene oxides with phenol and organic acids, alkyl aryl sulfonates, complex ether alcohols, quaternary ammonium compounds, and the like.

In the preparation of wettable powder or dust compositions, the active ingredient is dispersed in and on an appropriately divided solid carrier such as clay, talc, bentonite, diatomaceous earth, fuller's earth, and the like. In the formulation of the wettable powders, the aforementioned dispersing agents as well as lignosulfonates can be included.

The required amount of the active ingredient contemplated herein can be applied per acre treated in from 1 to 200 gallons or more of liquid carrier and/or diluent or in from about 5 to 500 pounds of inert solid carrier and/or diluent. The concentration in the liquid concentrate will usually vary from about 5 to 95 percent by weight and in the solid formulations from about 0.5 to about 90 percent by weight. Satisfactory sprays or dusts for general use contain from about 0.001 to about 100 pounds of active ingredient per acre, preferably from about 0.01 to about 15 pounds of active ingredient per acre, and more preferably from about 0.1 to about 5 pounds of active ingredient per acre.

Formulations useful in the conduct of this invention can also contain other optional ingredients such as stabilizers or other biologically active compounds, insofar as they do not impair or reduce the activity of the active ingredient and do not harm the plant being treated. Other biologically active compounds include, for example, one or more insecticidal, herbicidal, fungicidal, nematicidal, miticidal, plant growth regulators or other known compounds. Such combinations can be used for the known or other purpose of each ingredient and may provide a synergistic effect.

The cyclopropylmalonanilate derivative compounds of formula 1 are preferably applied to plants under substantially average or normal growing conditions. The cyclopropylmalonanilate derivative compounds used in this invention may be applied during the plant vegetative growth phase or the plant reproductive growth phase to obtain plant growth retardation.

Such compounds are useful in agriculture, horticulture and related fields and can be applied in general to both gymnosperms and angiosperms, in particular, to vegetation such as woody plants and turfgrasses to retard plant growth. The compounds are useful, for example, in controlling the height of vegetation in right-of-way areas and for growth retardation following pruning of trees and shrubs and the like with no adverse ecological effect.

As used herein, plants refer in general to any agronomic or horticultural plants, woody plants, ornamentals and turfgrasses. Illustrative of woody plants which can be treated by the cyclopropylmalonanilate derivative compounds of formula 1 according to the method of this invention include, for example, red maple, sycamore, red oak, American elm, linden, ginkgo, oaks, ashes, maples, apple trees, Chinese elm, crabapples, Russian olive, silver maple, sugar maple, water oak, poplars, conifers and the like. Illustrative of other plants which can be treated by the compounds of formula 1 according to the method of this invention include, for example, corn, cotton, sweet potatoes, white potatoes, alfalfa, wheat, rye, rice, barley, oats, sorghum, dry beans, soybeans, sugar beets, sunflowers, tobacco, tomatoes, canola, deciduous fruit, citrus fruit, tea, coffee, olives, pineapple, cocoa, banana, sugar cane, oil palm, herbaceous bedding plants, woody shrubs, turfgrasses, ornamental plants, evergreens, trees, flowers, and the like.

The cyclopropylmalonanilate derivative compounds contemplated herein are effective in retarding plant growth. Such compounds have a high margin of safety in that when used in sufficient amount to provide a growth redardation effect, they do not burn or injure the plant, and they resist weathering which includes wash-off caused by rain, decomposition by ultraviolet light, oxidation, or hydrolysis in the presence of moisture or, at least, such decomposition, oxidation, and hydrolysis as would materially decrease the desirable plant growth retardant characteristic of the active ingredient or impart undesirable characteristics, for instance, phytotoxicity, to the active ingredients. Mixtures of the active compounds can be employed if desired as well as combinations of the active compounds with other biologically active compounds or ingredients as indicated above.

This invention is illustrated by the following examples.

## Example I

### Preparation of ethyl 1-(2-methyl-4,5-dichlorophenylaminocarbonyl)cyclopropanecarboxylate

Into a nitrogen-purged round bottom flask was charged 5.53 grams (0.03 mole) of 2-methyl-4,5-dichloroaniline, 3.18 grams (0.03 mole) of triethylamine and 190 milliliters of tetrahydrofuran solvent. With vigorous stirring, a 5.55 gram (0.03 mole) portion of ethyl 1-chlorocarbonylcyclopropanecarboxylate prepared in Example V was added in one portion, after which the mixture was stirred at ambient temperature for a six-hour period. A precipitate of triethylamine hydrochloride was then filtered off and the filtrate vacuum stripped to give a light yellow solid. The solid was taken up in ether and the solution water-washed, dried over magnesium sulfate, and solvent evaporated to give a yellow powder. Recrystallization from ethyl acetate-hexane gave 4.51 grams (0.01 mole) of ethyl 1-(2-methyl-4,5-dichlorophenylaminocarbonyl)-cyclopropanecarboxylate having a melting point of 105°C-107°C. Elemental analysis of the product indicated the following:

| Analysis: $C_{14}H_{15}Cl_2NO_3$ | | | |
|---|---|---|---|
| Calculated: | C, 53.18; | H, 4.78; | N, 4.43 |
| Found: | C, 53.41; | H, 4.76; | N, 4.44 |

This compound is referred to hereinafter as Compound 77.

Example II

In a manner similar to that employed in Example I, other compounds were prepared. The structures and analytical data for Compounds 78 through 96 are set forth in Table A below.

## TABLE A

**Representative Malonic Acid Derivative Compounds**

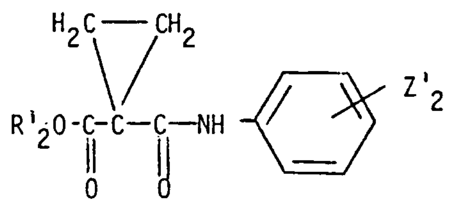

| Substituents | | | Elemental Analysis | | | | | | |
|---|---|---|---|---|---|---|---|---|---|
| | | | Calculated | | | Found | | | Melting Point |
| Compound No. | R'$_2$ | Z'$_2$ | C | H | N | C | H | N | °C |
| 78 | $C_2H_5$ | 2,4,5-$Cl_3$ | 46.38 | 3.59 | 4.16 | 46.69 | 3.99 | 4.10 | 130-132.5 |
| 79 | $C_2H_5$ | 3,4-$Cl_2$ | 51.67 | 4.34 | 4.64 | 51.95 | 4.34 | 4.72 | 107-110 |
| 80 | $C_2H_5$ | 2,4-$Cl_2$ | 51.67 | 4.34 | 4.64 | 51.27 | 4.53 | 4.46 | 95-98 |
| 81 | $C_2H_5$ | 2,5-$Cl_2$ | 51.67 | 4.34 | 4.64 | 51.36 | 4.48 | 4.49 | 105-108 |
| 82 | $C_2H_5$ | 2-F-4-Cl | 54.65 | 4.59 | 4.90 | 54.92 | 4.71 | 4.85 | 94.5-96 |
| 83 | $C_2H_5$ | 4-Cl | 58.32 | 5.27 | 5.23 | 58.15 | 5.29 | 5.16 | 91-93 |
| 84 | $C_2H_5$ | 4-Br | 50.02 | 4.52 | 4.49 | 50.18 | 4.69 | 4.52 | 92.5-95 |
| 85 | $C_2H_5$ | 3,4-$Br_2$ | 39.92 | 3.35 | 3.58 | 40.18 | 3.47 | 3.60 | 128-130 |
| 86 | $C_2H_5$ | 3,5-$BR_2$ | 39.92 | 3.35 | 3.58 | 39.82 | 3.32 | 3.46 | 91-92.5 |
| 87 | $C_2H_5$ | 2,4-$Br_2$ | 39.92 | 3.35 | 3.58 | 40.02 | 3.61 | 3.11 | 102-103.5 |
| 88 | $C_2H_5$ | 2-Cl-4-Br | 45.04 | 3.78 | 4.04 | 45.28 | 3.98 | 3.90 | 109-110.5 |
| 89 | $C_2H_5$ | 2-Br-4-Cl | 45.04 | 3.78 | 4.04 | 44.89 | 4.29 | 3.80 | 95-96 |
| 90 | $C_2H_5$ | 3-Cl-4-Br | 45.04 | 3.78 | 4.04 | 45.16 | 4.20 | 3.79 | 113-116 |
| 91 | $C_2H_5$ | 2-$CH_3$-4-Br-5-Cl | 46.62 | 4.19 | 3.88 | 48.14 | 4.74 | 3.86 | 119-121 |
| 92 | $C_2H_5$ | 2-F-4-Br | 47.29 | 3.97 | 4.24 | 46.87 | 4.07 | 4.02 | 102-103 |
| 93 | $C_2H_5$ | H | 66.83 | 6.47 | 6.00 | 66.54 | 6.48 | 5.80 | 85-89 |
| 94 | $C_2H_5$ | 3,5-$Cl_2$ | 51.67 | 4.34 | 4.64 | 51.52 | 4.52 | 4.36 | 64-67 |
| 95 | $C_2H_5$ | 4-CN | 65.10 | 5.46 | 10.85 | 65.02 | 5.51 | 10.67 | 129-132 |
| 96 | $C_2H_5$ | 2-$CH_3$-4-Br | 51.55 | 4.94 | 4.29 | 51.72 | 4.74 | 4.31 | 89-91 |

Example III

Preparation of 1-(2-methyl-4,5-dichlorophenylaminocarbonyl)cyclopropanecarboxylic acid

A solution containing 0.34 gram (0.006 mole) of potassium hydroxide and 0.109 gram (0.006 mole) of water in 80 milliliters of ethanol was prepared in a 250 milliliter round bottom flask. With cooling to a temperature of 0°C in an ice/NaCl bath and stirring, a solution of ethyl 1-(2-methyl-4,5-dichlorophenylaminocarbonyl)cyclopropanecarboxylate prepared in Example I in a small volume of ethanol was added and the mixture allowed to stir with warming to room temperature over a 72 hour period. The mixture was vacuum evaporated to give a white solid residue which was dissolved in water and extracted twice with ether. The ether extracts were discarded. The water solution was acidified to a pH of 2 with 25% HCl solution causing separation of a solid which was taken up into ether, and the acidified aqueous phase was extracted four times. The combined ether extracts were dried over magnesium sulfate and vacuum evaporated to give a white solid. This white solid was water-washed and dried in a vacuum oven to give 1.85 grams (0.006 mole) of 1-(2-methyl-4,5-dichlorophenylaminocarbonyl)cyclopropanecarboxylicacid having a melting point of 248°C-251°C. Elemental analysis of the product indicated the following:

| Analysis: $C_{12}H_{11}Cl_2NO_3$ | | | |
|---|---|---|---|
| Calculated: | C, 50.02; | H, 3.85; | N, 4.86 |
| Found: | C, 50.51; | H, 4.31; | N, 4.83 |

This compound is referred to hereinafter as Compound 110.

Example IV

In a manner similar to that employed in Example III, other compounds were prepared. The structures and analytical data for Compounds 111 through 128 are set forth in Table B below.

TABLE B

Representative Malonic Acid Derivative Compounds

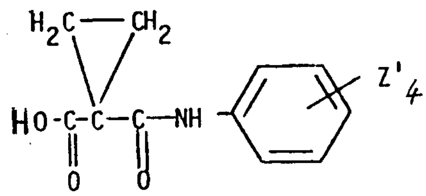

| Substituents | | Elemental Analysis | | | | | | |
|---|---|---|---|---|---|---|---|---|
| | | Calculated | | | Found | | | Melting Point |
| Compound No. | Z'4 | C | H | N | C | H | N | °C |
| 111 | 2-$CH_3$-4-Br | 48.34 | 4.06 | 4.70 | 48.20 | 4.06 | 4.66 | 204.5-206 |
| 112 | 2,4,5-$Cl_3$ | 42.82 | 2.61 | 4.54 | 43.11 | 3.14 | 4.42 | 250 |
| 113 | 2,5-$Cl_2$ | 48.20 | 3.31 | 5.11 | 48.33 | 3.26 | 4.96 | 223.5-226 |
| 114 | 2,4-$Cl_2$ | 48.20 | 3.31 | 5.11 | 45.26 | 3.40 | 5.03 | 189-190 |
| 115 | 2-F-4-Cl | 51.27 | 3.52 | 5.44 | 51.18 | 3.70 | 5.22 | 202-204 |
| 116 | 4-Cl | 55.12 | 4.21 | 5.84 | 54.69 | 4.35 | 5.59 | 217-219 |
| 117 | 4-Br | 46.50 | 3.55 | 4.93 | 46.36 | 3.45 | 4.86 | 220-222 |
| 118 | 3,4-$Br_2$ | 36.39 | 2.50 | 3.86 | 37.13 | 2.70 | 3.83 | 224-226.5 |
| 119 | 3,5-$Br_2$ | 36.39 | 2.50 | 3.86 | 36.99 | 2.60 | 3.82 | 211-212 |
| 120 | 2,4-$Br_2$ | 36.39 | 2.50 | 3.86 | 36.61 | 2.95 | 4.04 | 222-225 |
| 121 | 2-Cl-4-Br | 41.47 | 2.85 | 4.40 | 39.74 | 3.90 | 3.95 | 166-168 (dec.) |
| 122 | 2-Br-4-Cl | 41.47 | 2.85 | 4.40 | 41.67 | 3.28 | 3.91 | 210-211 |
| 123 | 3-Cl-4-Br | 41.47 | 2.85 | 4.40 | 41.70 | 3.23 | 4.11 | 211-214 |
| 124 | 2-$CH_3$-4-Br-5-Cl | 43.33 | 3.33 | 4.21 | 45.47 | 4.08 | 3.91 | 231-234 |
| 125 | 2-F-4-Br | 43.73 | 3.00 | 4.64 | 43.97 | 3.05 | 4.30 | 203.5-207 |
| 126 | 4-$CF_3$ | 52.75 | 3.69 | 5.13 | 52.73 | 3.90 | 5.04 | 195-196.5 |
| 127 | 3,5-$Cl_2$ | NMR ($CDCl_3$): 1.52 (S,4H), 7.02-7.74 (m,4H) 10.08(S, H) ppm. | | | | | | 198-202 |
| 128 | 3,4-$Cl_2$ | 48.20 | 3.31 | 5.11 | 48.79 | 3.80 | 5.26 | 220-222.5 |

EXAMPLE V

Preparation of ethyl 1-chlorocarbonyl-cyclopropanecarboxylate

Into a stirred solution containing 15.1 grams (0.27 mole) of potassium hydroxide In 240 milliliters of ethanol and 4.83 grams (0.27 mole) of water was added dropwise, with cooling at a temperature of 0 °C, 50.0 grams (0.27 mole) of diethyl 1,1-cyclopropanedicarboxylate. The mixture was stirred for about 16 hours at room temperature. Solvent was removed under reduced pressure to give a white residue which was dissolved in water and extracted with ether. The water solution was acidified to a pH of 2 with 25% aqueous hydrochloric acid and the organic acid was extracted from the aqueous suspension with ethyl ether (4 x 400 milliliters). The ether extract was dried over magnesium sulfate and vacuum stripped to give the monocarboxylic acid as a clear liquid. The clear liquid was dissolved in 300 milliliters of methylene chloride after which 74 grams (0.62 mole) of thionyl chloride were added, and the resulting mixture was then heated under reflux for approximately 16 hours. Volatiles were removed under reduced pressure to give 45.7 grams (0.25 mole) of ethyl 1-chlorocarbonylcyclopropanecarboxylate. NMR analysis of the product indicated the following:

NMR ($CDCl_3$): δ 1.22-1.50 (t, 3H), 1.75 (s, 4H), 4.1-4.52 (q, 2H) ppm.

This compound is referred to hereinafter as Compound 155.

EXAMPLE VI

Effect of Representative cyclopropylmalonanilate Derivative Compounds on Plant Growth Retardation-Snapbeans and Wheat

Solutions of the test compounds identified in Table C below were prepared by dissolving 68.8 milligrams of the particular compound in 5.5 milliliters of acetone and then adding water to a final volume of 11.0 milliliters. If clouding of the solution occurred as the water was added, the use of water was discontinued and acetone was added to a final volume of 1.0 milliliters. The resulting stock solutions contained 6255 parts per million by weight of the particular compound. the test concentration in parts of the test compound per million parts by weight of final solution employed in the growth retardation tests in Table C were obtained by appropriate dilutions of the stock suspension with acetone and water (50/50 volume/volume).

Seeds of snapbeans, wheat, velvetleaf, cucumber, sunflower, flax, buckwheat, tomato, perennial rye, marigold, soybean, barnyard grass, wild oats and pea were planted in a sandy loam soil in a flat having the following dimensions: 3.5 inches (i.e. 8,89 cm) in width x 7.9 inches (i.e. 20 cm) in lenght x 1.0 inches (i.e. 2,54 cm) in height. Twelve to fourteen days after planting at the time the first trifoliolate leaf of snapbean is at least 3.0 centimeters long, each concentration of the test compounds identified in Table C was applied to one flat as a foliar spray by use of an aspirated spray apparatus set at 10 psig (i.e. 78,9. $10^3$ Pa) air pressure (all flats sprayed at a rate of 4 pounds of active ingredient i.e. 4,48 kg/ha per acre). As a control, a water-acetone solution containing no test compound was also sprayed on a flat. When dry, all of the flats of plants were placed in a greenhouse at a temperature of 80 °F ± 5 °F and humidity of 50 percent ± 5 percent. Visual indications of growth retardation activity were observed and recorded 10 to 14 days after treatment.

Visual observations of growth retardation were recorded employing a system of numerical ratings. Numerical ratings from "0" to "10" were used to designate the degree of growth retardation activity observed in comparison with the untreated control. A "0" rating indicates no visible response, a "5" rating indicates 50 percent more growth retardation in comparison with the control, and a "10" rating indicates 100 percent more growth retardation in comparison with the control. Stated in a similar way, a "5" rating indicates that the increment in plant growth is only half that of the control or that the plant has increased in growth at half the rate of the control. This rating system indicates any retardation of plant height as compared to the untreated control. The results are reported in Table C.

## TABLE C

### Effect of Representative Active Compounds on Plant Growth Retardation Snapbeans

| Compound No. | Growth Retardation Rating |
| --- | --- |
| Control | 0 |
| 78 | 2 |
| 79 | 5 |
| 80 | 2 |
| 81 | 2 |
| 82 | 3 |
| 83 | 2 |
| 84 | 4 |
| 85 | 2 |
| 86 | 2 |
| 87 | 2 |
| 88 | 3 |
| 90 | 2 |
| 92 | 4 |
| 94 | 2 |
| 95 | 3 |
| 110 | 4 |
| 111 | 9 |
| 112 | 3 |
| 114 | 4 |
| 115 | 3 |
| 116 | 7 |
| 117 | 2 |
| 118 | 2 |
| 120 | 2 |

## TABLE C (Continued)

### Effect of Representative Active Compounds on Plant Growth Retardation Snapbeans

| Compound No. | Growth Retardation Rating |
|---|---|
| 121 | 4 |
| 122 | 4 |
| 123 | 3 |
| 125 | 5 |
| 126 | 4 |
| 128 | 2 |

## TABLE C

### Effect of Representative Active Compounds on Plant Growth Retardation Wheat

| Compound No. | Growth Retardation Rating |
|---|---|
| Control | 0 |
| 82 | 2 |
| 84 | 2 |
| 88 | 2 |
| 92 | 3 |
| 110 | 2 |
| 111 | 3 |
| 114 | 4 |
| 115 | 5 |
| 116 | 2 |
| 117 | 2 |
| 121 | 2 |
| 122 | 2 |
| 123 | 2 |
| 125 | 2 |
| 126 | 2 |

The results in Table C demonstrate that treatment of plants with cyclopropylmalonanilate derivative compounds provides significant growth retardation in comparison with untreated control plants.

## EXAMPLE VII

### Effect of Representative cyclopropylmalonanilate Derivative Compounds on Plant Growth Retardation-Wheat

Solutions of the test compounds identified in Table D below were prepared by dissolving the compounds in acetone/water (50:50 volume/volume) containing 0.05 percent volume/volume of Triton X-100 surfactant commercially available from Rhom and Haas Company, Philadelphia, Pennsylvania. As detailed below, these solutions of test compounds were applied to wheat at a concentration of 0.5 pounds of active ingredient per acre (i.e. 0,56 kg/ha) or 1.0 pounds of active ingredient per acre (i.e. 1,12 kg/ha).

Wheat seeds were planted in a sandy loam soil in a flat having the following dimensions : 3.5 inches in width x 7.9 inches in length x 1.0 inches in height. Eight days after emergence at the 2-3 leaf growth stage of wheat, each concentration of the test compounds identified in Table D was applied to one flat as a foliar spray by use of an aspirated spray apparatus set at 10 psig air pressure (all flats sprayed at a volume of 120 gallons per acre i.e. 1122 l/ha).

As a control, a water-acetone solution containing no test compound was also sprayed on a flat. When dry, all of the flats of wheat were placed in a greenhouse at a temperature of 80°F + 5°F (i.e. 27°C + 3°C) and humidity of 50 percent + 5 percent. Visual indications of growth retardation activity were observed and recorded 14 days after treatment.

Visual observations of growth retardation were recorded employing a system of percentage ratings. These percentage ratings from 0 to 100 were used to designate the degree of growth retardation activity observed in comparison with the untreated control. A 0 percent rating indicates no visible response, a 50 percent rating indicates that the increment in wheat growth is only half that of the control or that wheat has increased in growth at half the rate of the control and a 100 percent rating indicates a maximum response. This rating system indicates any retardation of wheat height as compared to the untreated control. The results are reported in Table D.

TABLE D

| Effect of Representative Active Compounds on Plant Growth Retardation - Wheat | | |
|---|---|---|
| Compound No. | Rate (Pounds/Acre) | Percent Growth Retardation |
| Control | -- | 0 |
| Compound 96 | 0.5<br>1.0 | 30<br>40 |
| Compound 111 | 0.5<br>1.0 | 60<br>70 |
| Compound 114 | 0.5<br>1.0 | 20<br>30 |
| Compound 82 | 0.5<br>1.0 | 10<br>10 |
| Compound 115 | 0.5<br>1.0 | 30<br>40 |
| Compound 116 | 0.5<br>1.0 | 40<br>70 |
| Compound 117 | 0.5<br>1.0 | 50<br>60 |

The results in Table D demonstrate that treatment of wheat with cyclopropylmalonanilate derivative compounds provides signifcant growth retardation in comparison with untreated control wheat.

EXAMPLE VIII

Effect of Representative Cyclopropylmalonanilate Derivative Compounds on Plant Growth Retardation-Red Maple and Sycamore

Solutions of the test compounds identified in Table E below were prepared by dissolving the compounds in acetone/water (50 : 50 volume/volume) containing 0.1 percent volume/volume of Triton X-100 surfactant commercially available from Rhom and Haas Company, Philadelphia, Pennsylvania. As detailed below, these solutions of test compounds were applied to red maple and sycamore at a concentration of 1.0, 2.0 or 4.0 pounds of active ingredient per acre (i.e. 1.12 ; 2.24 ; or 4.48kg/ha).

Bare-root seedlings of red maple (Acer rubrum) and sycamore (Platanus occidentalis) were obtained commercially and grown in one gallon plastic containers containing a sandy loam soil.
The seedlings were maintained in a greenhouse at a temperature of 80°F + 5°F (i.e. 27°C + 3°C) and humidity of 50 + 5 percent. After a period of one month, the developing trees were disbudded to one main dominant shoot 4-6 inches (i.e. 10, 16 - 15,24 cm) in length. At this time, each concentration of the test compounds identified in Table E was applied to separate trees as a foliar spray by use of an aspirated spray apparatus set at 10 psig (i.e. $78{,}9.10^3$ Pa) air pressure (all trees sprayed at a volume of 120 gallons per acre i.e. 1122 l/ha). As a control, a water-acetone solution containing no test compound was also sprayed on certain trees. When dry, all of the trees were returned to the greenhouse for a period of one month. Measured indications of growth retardation activity were observed and recorded at this time (one month after treatment).

The percent retardation of shoot elongation in Table E was determined by actual measurement of the shoot of each tree which was compared to the untreated control. The average shoot length of the untreated control trees was 48 centimeters for red maple and 53 centimeters for sycamore. the results in Table E represent the average of 3 repetitions.

TABLE E

| Effect of Representative Active Compounds on Plant Growth Retardation - Red Maple and Sycamore | | | |
|---|---|---|---|
| Compound No. | Rate (Pounds per Acre) | Percent Retardation of Shoot Elongation | |
| | | Red Maple | Sycamore |
| Control | -- | 0 | 0 |
| Compound 96 | 1.0<br>2.0<br>4.0 | 34<br>40<br>43 | 15<br>16<br>20 |
| Compound 111 | 1.0<br>2.0<br>4.0 | 76<br>97<br>97 | 77<br>84<br>88 |

The results in Table E demonstrate that treatment of red maple and sycamore with cyclopropylmalonanilate derivative compounds provides significant growth retardation in comparison with untreated control red maple and sycamore.

Example XIX

Effect of Representative Cyclopropylmalonanilate Derivative Compounds on Plant Growth Retardation-Red Maple and Sycamore

Solutions of the test compounds identified in Table F below were prepared by dissolving the compounds in acetone/water (50:50 volume/volume) containing 0.1 percent volume/volume of Triton X-100 surfactant commercially available from Rhom and Haas Company, Philadelphia, Pennsylvania. As detailed below, these solutions of test compounds were applied to red maple and sycamore at a concentration of 1.0 or 2.0 pounds of active ingredient per acre.

Bare-root seedlings of red maple (Acer rubrum) and sycamore (Platanus occidentalis) were obtained commercially and grown in one gallon plastic containers containing a sandy loam soil. The seedlings were

maintained in a greenhouse at a temperature of 80 °F ± 5 °F and humidity of 50 ± 5 percent. After a period of 3 months, the developing trees were pruned to a 50 percent reduction in height. At 24 days after pruning, each concentration of the test compounds identified in Table F was applied to separate trees as a foliar spray by use of an aspirated spray apparatus set at 10 psig air pressure (all trees sprayed at a volume of 120 gallons per acre). As a control, a water-acetone solution containing no test compound was also sprayed on certain trees. When dry, all of the trees were returned to the greenhouse for a period of 45 days. Visual indications of growth retardation activity were observed and recorded at this time (45 days after treatment).

The percent retardation of regrowth in Table F was determined by visual observation of the regrowth of each tree in comparison with the untreated control. A 0 percent rating indicates no visible response, a 50 percent rating indicates that the increment in tree growth is only half that of the control or that the tree has increased in growth at half the rate of the control, and a 100 percent rating indicates a maximum response. This rating system indicates any retardation of regrowth as compared with the untreated control. The results in Table F represent the average of 3 repetitions.

TABLE F

| Effect of Representative Active Compounds on Plant Growth Retardation - Red Maple and Sycamore | | | |
|---|---|---|---|
| Compound No. | Rate (Pounds per Acre) | Percent Retardation of Regrowth | |
| | | Red Maple | Sycamore |
| Control | -- | 0 | 0 |
| Compound 96 | 1.0<br>2.0 | 47<br>42 | 8<br>9 |
| Compound 111 | 1.0<br>2.0 | 85<br>91 | 43<br>46 |

The results in Table F demonstrate that treatment of red maple and sycamore with cyclopropylmalonanilate derivative compounds provides significant retardation of regrowth in comparison with untreated control red maple and sycamore.

**Claims**

**1.** A cyclopropylmalonanilate derivative compound having the formula:

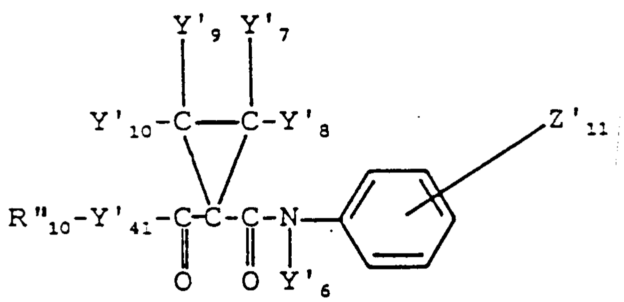

wherein:

$Z'_{11}$ is the same or different and is one or more hydrogen, halogen, haloalkyl, polyhaloalkyl, polyhaloalkoxy, alkyl, alkoxy, alkylthio, alkylsulfonyl, alkylsulfinyl, aryl, aryloxy, arylthio, arylsulfonyl, nitro, cyano, dialkoxyphosphinyl, alkanoyl, aroyl, alkoxycarbonyl, alkoxycarbonylalkyl, alkanoylamino, sulfonylamino, alkylsulfonylamino, alkanoyloxy, alkenyl or -CH = CH-CH = CH-;

$Y'_6$ is hydrogen or alkyl;

$Y'_7$, $Y'_8$, $Y'_9$ and $Y'_{10}$ are independently hydrogen, halogen or alkyl;

$Y'_{41}$ is 0, S, or NH ;

$R''_{10}$ is hydrogen, ammonium, alkylammonium, polyalkylammonium, hydroxyalkylammonium, poly-(hydroxyalkyl)ammonium, an alkali metal or alkaline earth metal or alkyl, hydroxyalkyl, alkoxyalkyl,

alkoxycarbonylalkyl, alkylaminoalkyl, dialkylaminoalkyl, aryl, mercaptoalkyl, alkylthioalkyl, arylthioalkyl, aryloxyalkyl, alkylsulfonylalkyl, alkylsulfinylalkyl, alkanoylalkyl, aroylalkyl, dialkoxyphosphinylalkyl, diaryloxyphosphinylalkyl, hydroxyalkylthioalkyl, hydroxyalkylsulfonylalkyl, alkoxyalkylthioalkyl, alkoxyalkylsulfonylalkyl, poly(oxyalkylene)alkyl, cyanoalkyl, nitroalkyl, alkylideneamino, carbamoylalkyl, alkylcarbamoylalkyl, dialkylcarbamoylalkyl, aminoalkyl, alkanoylaminoalkyl, alkanoyloxyalkyl, alkoxycarbonylaminoalkyl, cyanoaminoalkyl, carbamoyloxyalkyl, alkylcarbamoyloxyalkyl, dialkylcarbamoyloxyalkyl, alkoxycarbonyloxyalkyl, alkoxycarbonylthioalkyl, aminosulfonylalkyl, alkylaminosulfonylalkyl or dialkylaminosulfonylalkyl.

**2.** A cyclopropylmalonanilate derivative compound of claim 1 having one of the formulae chosen among:

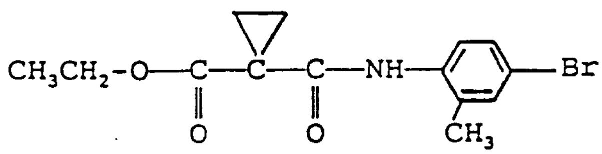

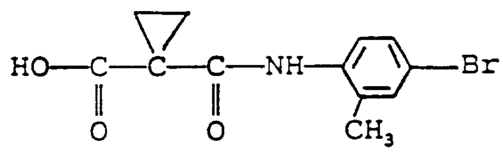

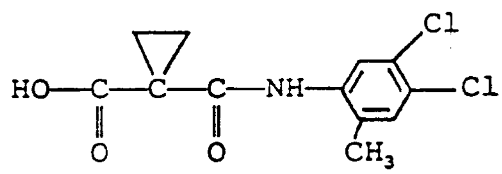

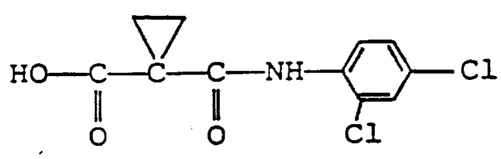

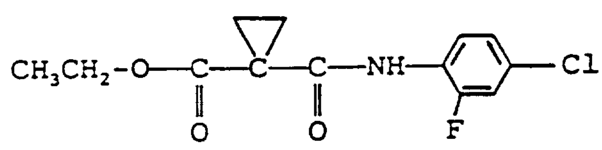

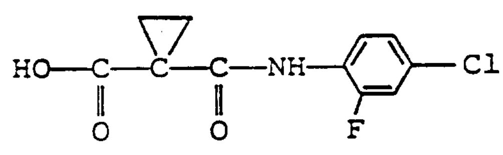

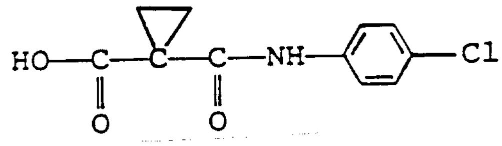

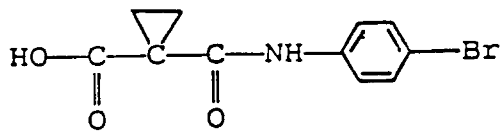

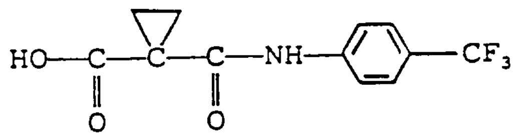

3. A cyclopropylmalonanilate derivative compound of claim 1 or 2 wherein said compound is a derivative salt.

4. A cyclopropylmalonanilate derivative compound of claim 3 wherein the derivative salt is selected from an alkali metal, an alkaline earth metal, ammonium, alkylammonium, polyalkylammonium, hydroxyalkylammonium, poly(hydroxyalkyl) ammonium or mixtures thereof.

5. A composition for retarding plant growth comprising an acceptable carrier and an effective amount, sufficient to retard plant growth, of an active compound 1 according to anyone of claim 1 to 4.

**Patentansprüche**

1. Cyclopropylmalonanilatderivat-Verbindung der Formel:

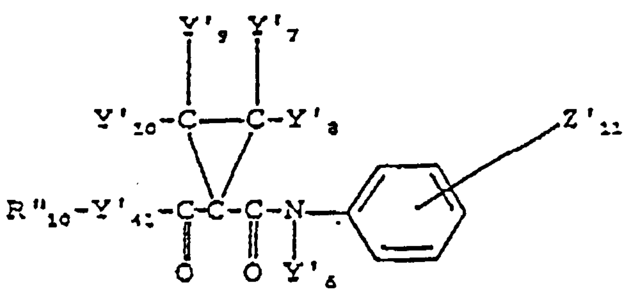

worin

$Z'_{11}$, das gleich oder verschieden sein kann, eine oder mehrere der Gruppen: Wasserstoff, Halogen, Halogenalkyl, Polyhalogenalkyl, Polyhalogenalkoxy, Alkyl, Alkoxy, Alkylthio, Alkylsulfonyl, Alkylsulfinyl, Aryl, Aryloxy, Arylthio, Arylsulfonyl, Nitro, Cyano, Dialkoxyphosphinyl, Alkanoyl, Aroyl, Alkoxycarbonyl, Alkoxycarbonylalkyl, Alkanoylamino, Sulfonylamino, Alkylsulfonylamino, Alkanoyloxy, Alkenyl oder -CH=CH-CH=CH- bedeutet;

$Y'_6$ Wasserstoff oder Alkyl ist;

$Y'_7$, $Y'_8$, $Y'_9$ und $Y'_{10}$ unabhängig voneinander Wasserstoff, Halogen oder Alkyl bedeuten;

$Y'_{41}$ ist O, S oder NH;

$R''_{10}$ ist Wasserstoff, Ammonium, Alkylammonium, Polyalkylammonium, Hydroxyalkylammonium, Poly-(hydroyalkyl)ammonium, ein Alkalimetall oder Erdalkalimetall oder Alkyl, Hydroxyalkyl, Alkoxyalkyl,

Alkoxycarbonylalkyl, Alkylaminoalkyl, Dialkylaminoalkyl, Aryl, Mercaptoalkyl, Alkylthioalkyl, Arylthioalkyl, Aryloxyalkyl, Alkylsulfonylalkyl, Alkylsulfinylalkyl, Alkanoylalkyl, Aroylalkyl, Dialkoxyphosphinylalkyl, Diaryloxyphosphinylalkyl, Hydroxyalkylthioalkyl, Hydroxyalkylsulfonylalkyl, Alkoxyalkylthioalkyl, Alkoxyalkylsulfonylalkyl, Poly(oxyalkylen)alkyl, Cyanoalkyl, Nitroalkyl, Alkylidenamino, Carbamoylalkyl, Alkylcarbamoylalkyl, Dialkylcarbamoylalkyl, Aminoalkyl, Alkanoylaminoalkyl, Alkanoyloxyalkyl, Alkoxycarbonylaminoalkyl, Cyanoaminoalkyl, Carbamoyloxyalkyl, Alkylcarbamoyloxyalkyl, Dialkylcarbamoyloxyalkyl, Alkoxycarbonyloxyalkyl, Alkoxycarbonylthioalkyl, Aminosulfonylalkyl, Alkylaminosulfonylalkyl oder Dialkylaminosulfonylalkyl.

**2.** Cyclopropylmalonanilatderivat-Verbindung nach Anspruch 1, die eine der folgenden Formeln aufweist:

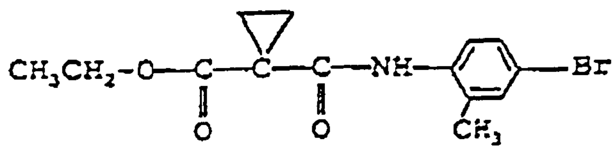

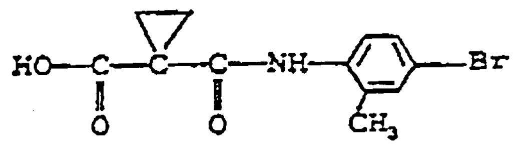

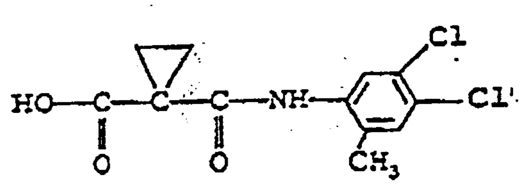

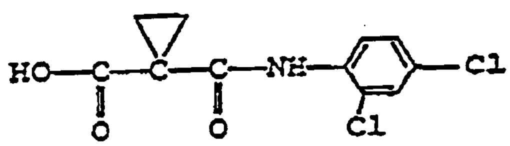

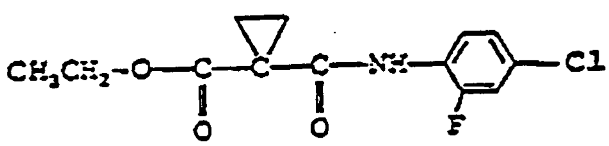

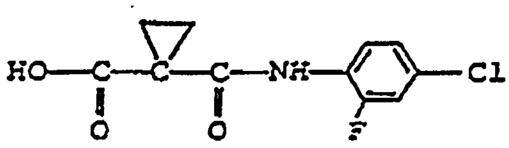

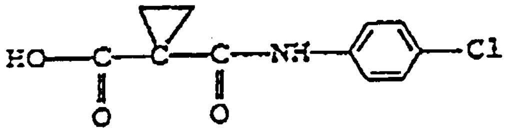

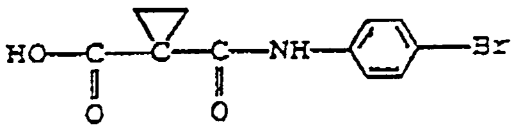

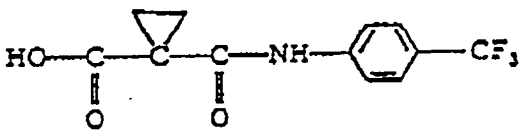

3. Cyclopropylmalonanilatderivat-Verbindung nach Anspruch 1 oder 2, worin die genannte Verbindung ein Salz-Derivat ist.

4. Cyclopropylmalonanilatderivat-Verbindung nach Anspruch 3, worin das Salz-Derivat ausgewählt wird aus einem Alkalimetall, einem Erdalkalimetall, Ammonium, Alkylammonium, Polyalkylammonium, Hydroxyalkylammonium, Poly(hydroxyalkyl)ammonium oder Mischungen von diesen.

5. Zusammensetzung zur Verzögerung von Pflanzenwachstum, die einen geeigneten Träger und eine wirksame Menge, die ausreicht, um Pflanzenwachstum zu verzögern, einer aktiven Verbindung 1 nach einem der Ansprüche 1 bis 4 umfaßt.

**Revendications**

1. Dérivé de cyclopropylmalonanilate, répondant à la formule :

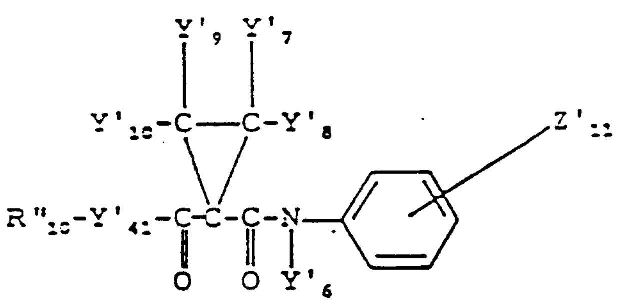

dans laquelle :

les groupes $Z'_{11}$ sont identiques ou différents et représentent un ou plusieurs atomes d'hydrogène, atomes d'halogènes, groupes halogénalkyle, polyhalogénalkyle, polyhalogénalkoxy, alkyle, alkoxy, alkylthio, alkylsulfonyle, alkylsulfinyle, aryle, aryloxy, arylthio, arylsulfonyle, nitro, cyano, dialkoxyphosphinyle, alcanoyle, aroyle, alkoxycarbonyle, alkoxycarbonylalkyle, alcanoylamino, sulfonylamino, alkylsulfonylamino, alcanoyloxy, alcényle ou -CH = CH-CH = CH- ;

$Y'_6$ représente l'hydrogène ou un groupe alkyle ;

$Y'_7$, $Y'_8$, $Y'_9$ et $Y'_{10}$ représentent, indépendamment, l'hydrogène, des atomes d'halogènes ou des groupes alkyle ;

$Y'_{41}$ représente O, S ou un groupe NH ;

$R''_{10}$ représente l'hydrogène, un ion ammonium, alkylammonium, polyalkylammonium, hydroxyalkylammonium, poly(hydroxyalkyl)ammonium, un métal alcalin ou un métal alcalino-terreux ou un groupe alkyle, hydroxyalkyle, alkoxyalkyle, alkoxycarbonylalkyle, alkylaminoalkyle, dialkylaminoalkyle, aryle, mercaptoalkyle, alkylthioalkyle, arylthioalkyle, aryloxyalkyle, alkylsulfonylalkyle, alkylsulfinylalkyle, alcanoylalkyle, aroylalkyle, dialkoxyphosphinylalkyle, diaryloxyphosphinylalkyle, hydroxyalkylthioalkyle, hydroxyalkylsulfonylalkyle, alkoxyalkylthioalkyle, alkoxyalkylsulfonylalkyle, poly(oxyalkylène)alkyle, cyanalkyle, nitroalkyle, alkylidèneamino, carbamoylalkyle, alkylcarbamoylalkyle, dialkylcarbamoylalkyle, aminoalkyle, alcanoylaminoalkyle, alcanoyloxyalkyle, alkoxycarbonylaminoalkyle, cyanaminoalkyle, carbamoyloxyalkyle, alkylcarbamoyloxyalkyle, dialkylcarbamoyloxyalkyle, alkoxycarbonyloxyalkyle, alkoxycarbonylthioalkyle, aminosulfonylalkyle, alkylaminosulfonylalkyle ou dialkylaminosulfonylalkyle.

**2.** Dérivé de cyclopropylmalonanilate suivant la revendication 1, répondant à l'une des formules suivantes :

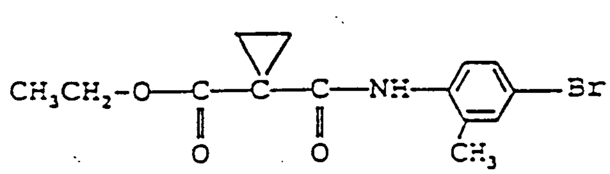

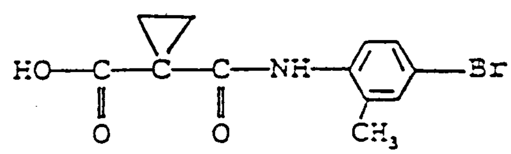

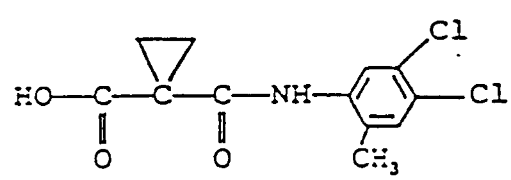

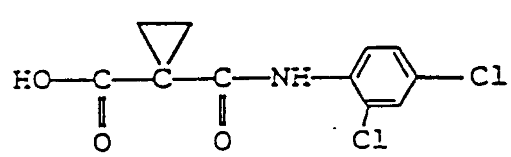

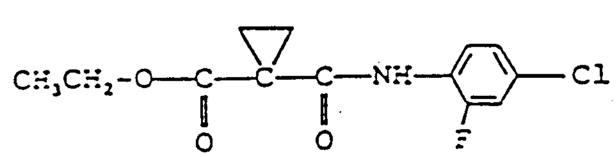

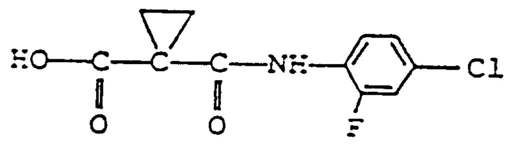

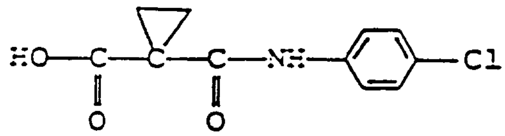

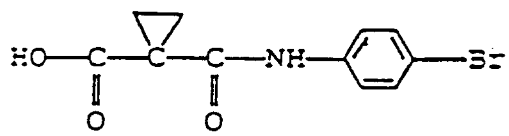

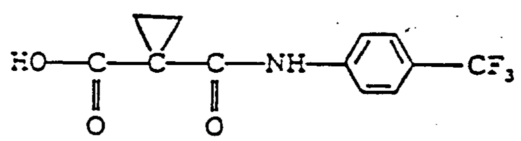

**3.** Dérivé de cyclopropylmalonanilate suivant la revendication 1 ou 2, qui consiste en un sel.

**4.** Dérivé de cyclopropylmalonanilate suivant la revendication 3, dans lequel le sel est choisi entre un sel de métal alcalin, un sel de métal alcalino-terreux, un sel d'ammonium, un sel d'alkylammonium, un sel de polyalkylammonium, un sel d'hydroxyalkylammonium, un sel de poly(hydroxyalkyl)ammonium et leurs mélanges.

**5.** Composition destinée à retarder la croissance de plantes, comprenant un support acceptable et une quantité efficace, suffisante pour retarder la croissance de plantes, d'un composé actif suivant l'une quelconque des revendications 1 à 4.